(19) **Europäisches Patentamt / European Patent Office / Office européen des brevets**

(11) **EP 2 425 818 A1**

(12) **EUROPEAN PATENT APPLICATION**
published in accordance with Art. 153(4) EPC

(43) Date of publication:
**07.03.2012 Bulletin 2012/10**

(21) Application number: **10769214.7**

(22) Date of filing: **27.04.2010**

(51) Int Cl.:
*A61K 9/107* (2006.01)  *A61K 31/4184* (2006.01)
*A61K 31/7068* (2006.01)  *A61P 35/00* (2006.01)

(86) International application number:
**PCT/CN2010/000577**

(87) International publication number:
**WO 2010/124525 (04.11.2010 Gazette 2010/44)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **27.04.2009  US 172901 P**

(71) Applicant: **Innopharmax, Inc.**
**Taipei, Taiwan 11492 (CN)**

(72) Inventors:
• **HSU, Chang-Shan**
**Taiwan 11492 (CN)**

• **HAO, Wei-Hua**
**Taiwan 11492 (CN)**
• **WANG, Jong-Jing**
**Taiwan 11492 (CN)**
• **LIN, Tsung-Hsin**
**Taiwan 11492 (CN)**

(74) Representative: **Becker Kurig Straus**
**Patentanwälte**
**Bavariastrasse 7**
**80336 München (DE)**

(54) **SELF MICRO-EMULSIFYING ORAL PHARMACEUTICAL COMPOSITION OF HYDROPHILIC DRUG AND PREPARATION METHOD THEREOF**

(57)  A self micro-emulsifying oral pharmaceutical composition of a hydrophilic drug or its pharmaceutical acceptable salt and the preparation method thereof are disclosed. The composition comprises hydrophilic drug such as bendamustine or gemcitabine, solvent, surfactant and hydrophilic carrier, and has good bioavailability and stability. Said hydrophilic carrier is compatible with drug solution and surfactant.

EP 2 425 818 A1

**Description**

FIELD OF THE INVENTION

The preset invention relates to an oral self micro-emulsifying pharmaceutical composition of hydrophilic drugs and methods for preparing the same.

BACKGROUND OF THE INVENTION

Oral administration is a convenient and user-friendly mode of drug administration, either in the form of a solid or a liquid suspension, which continues to dominate the area of drug delivery technologies. Even though many types of drugs could be administered orally with acceptable efficacy, there remains some problems for some classes of drugs, especially those which are known to have good solubility, but are extensively metabolized in the liver, easily pumped out by the intestinal epithelium (poor permeability) or irritative to the gastric mucosa, such as Class III drugs of Biopharmaceutics Classification System (BCS) provided by the U.S. Food and Drug Administration. Thus, for these drugs, injection administration become the major option to achieve acceptable drug absorption and bioavailability which however leads to increased risk and expenses and further is painful for patients.

A new technique called "self-emulsifying/microemulsifying drug delivery system (SEDDS/SMEDDS)" has been developed in the art which provides a good vehicle to improve bioavailability of hydrophobic drugs and make their oral delivery possible. Normally, the SEDDS/SMEDDS is composed of oil, a surfactant, a cosurfactant or solubilizer, and a hydrophobic drug. The underlying principle of said system is that when the SEDDS/SMEDDS contacts water, it spontaneously forms oil-in-water microemulsions under mild mechanical agitation. Consequently, a drug can be formulated so as to dissolve in a liquid-based formulation that does not contain an aqueous phase. It can then be filled into soft/hard capsules to form solid oral formulations. After being oral administered and contacting gastrointestinal fluids, said formulation is capable of self-emulsifying into microemulsions immediately so as to facilitate the dispersion, dissolution, stability and absorption of the drug, thus improving the bioavailability of said drug. However, for hydrophilic drugs, there are limitations to make them suitable in the SEDDS/SMEDDS. Other known strategies, e.g. liposomes, microparticles or prodrugs, have been reported to enhance the bioavailability of hydrophilic drugs, as described in, for example, U.S. Patent Nos. 7,220,428, 7,053,076, 7,217,735, and 7,309,696, and PCT Publication Nos. WO2004/0179 and WO2007/089043.

Therefore, there is still a need to develop an oral dosage form of hydrophilic drugs, especially an oral self-emulsifying pharmaceutical composition with good bioavailability and stability.

BRIEF SUMMARY OF THE INVENTION

In one aspect, the present invention provides an oral self micro-emulsifying pharmaceutical composition comprising:

(a) a therapeutically effective amount of a hydrophilic drug or its pharmaceutically acceptable salt;
(b) one or more solvents capable of dissolving the hydrophilic drug or its pharmaceutically acceptable salt to form a drug-solvent solution;
(c) a surfactant system comprising one or more surfactants, said surfactant system exhibiting a hydrophilic-lipophilic balance (HLB) value ranging from about 8 to about 17; and
(d) one or more hydrophilic carriers which are compatible with said drug-solvent solution and said surfactant system;

wherein the pharmaceutical composition is in a form of a self micro-emulsifying formulation for oral administration.

In another aspect, the invention provides a method of preparing the oral self micro-emulsifying pharmaceutical composition as set forth above, which comprise mixing together the hydrophilic drug or its pharmaceutically acceptable salt, the one or more solvents, the one or more hydrophilic carriers and the surfactant system to form the oral self micro-emulsifying pharmaceutical composition.

The various embodiments of the present invention are described in details below. Other characteristics of the present invention will be clearly presented by the following detailed description about the various embodiments and claims.

It is believed that a person of ordinary knowledge in the art where the present invention belongs can utilize the present invention to its broadest scope based on the description herein with no need of further illustration. Therefore, the following description should be understood as of demonstrative purpose instead of limitative in any way to the scope of the present invention.

BRIEF DESCRIPTION OF THE SEVERAL VIEWS OF THE DRAWINGS

For the purpose of illustrating the invention, there are shown in the drawings embodiments which are presently preferred. It should be understood, however, that the invention is not limited to the preferred embodiments shown.

In the drawings:

Figure 1 shows the profiles of plasma concentrations of gemcitabine after intravenous and oral administration.

Figure 2 shows the profiles of plasma concentrations of 2',2'-difluorodeoxyuridine (dFdU) after intravenous and oral administration.

DETAILED DESCRIPTION OF THE INVENTION

Unless otherwise defined herein, scientific and technical terms used in connection with the present invention shall have the meanings that are commonly understood by those of ordinary skill in the art. As used herein, the following terms have the meanings ascribed to them unless specified otherwise.

Unless otherwise required by context, singular terms shall include the plural and plural terms shall include the singular. The articles "a" and "an" are used herein to refer to one or more than one (*i.e.,* at least one) of the grammatical object of the article. By way of example, "an element" means one element or more than one element.

The present invention provides an oral self micro-emulsifying pharmaceutical composition of a hydrophilic drug which, in addition to the hydrophilic drug, one or more solvents for solving the hydrophilic drug to form a drug-solvent solution, and a surfactant system comprising one or more surfactants exhibiting a HLB value ranging from about 8 to about 17, comprises one or more hydrophilic carrier which are compatible with said drug-solvent solution and said surfactant system. The oral self-emulsifying pharmaceutical composition according to the invention exhibits excellent bioavailability of the drug through oral administration which is comparable to that of the drug through intravenous injection.

Accordingly, in one aspect, the present invention provides an oral self micro-emulsifying pharmaceutical composition comprising:

(a) a therapeutically effective amount of a hydrophilic drug or its pharmaceutically acceptable salt;
(b) one or more solvents capable of dissolving the hydrophilic drug or its pharmaceutically acceptable salt to form a drug-solvent solution;
(c) a surfactant system comprising one or more surfactants, said surfactant system exhibiting a hydrophilic-lipophilic balance (HLB) value ranging from about 8 to about 17; and
(d) one or more hydrophilic carriers which are compatible with said drug-solvent solution and said surfactant system;

wherein the pharmaceutical composition is in a form of a self micro-emulsifying formulation for oral administration..

As used herein, the term "self micro-emulsifying" is to describe a formulation which when contacting an aqueous medium (such as mixed with water) produces a fine oil-water emulsion. Particularly, the self micro-emulsifying pharmaceutical composition of the invention, when contacting an aqueous medium, forms an emulsion with a mean particle size of less than 800 nm, more particularly less than 400 nm, even more particularly less than 200 nm, and most particularly less than 100 nm. In one embodiment, the self micro-emulsifying pharmaceutical composition of the invention, when contacting an aqueous medium, forms an emulsion with a mean particle size of about 10 nm.

As used herein, the term "therapeutically effective amount" means a dose of the drug as used that is effective in exerting a therapeutic effect, particularly a dose of the drug which, after absorption into the body through the walls of gastrointestinal (GI) tract, yields a drug concentration in the blood effective in exerting a therapeutic effect on a target organ. Persons skilled in the art will understand that the amounts of the drug presented in the composition vary with the particular situation, including but not limited to, the species and dosage form of the drug and the size, age and condition of the subject.

According to the invention, the term "pharmaceutically acceptable salt" as used herein includes, but is not limited to, acid addition salts that substantially retain the biological effectiveness and properties of the free bases. Such acid addition salts may be formed with inorganic acids such as hydrochloric acid, hydrobromic acid, sulfuric acid, nitric acid, phosphoric acid and the like, and organic acids such as acetic acid, propionic acid, pyruvic acid, maleic acid, masonic acid, succinic acid, fumaric acid, tartaric acid, citric acid, benzoic acid, mandelic acid, methanesulfonic acid, ethanesulfonic acid, p-toluenesulfonic acid, salicylic acid, trifluoroacetic acid and the like.

According to the invention, the term "hydrophilic drug" as used herein refers to a drug that is the opposite of a lipophilic drug and exhibits a certain degree of solubility in aqueous medium. Particularly, the hydrophilic drug used in the invention has high solubility as defined in BCS (i.e. the highest dose is soluble in 250 ml or less of water over a range of pH from 1 to 7.5). Examples of the hydrophilic drug include but are not limited to albuterol, alendroate, amoxicillin, bendamustine, buspirone, calcitonin, captopril, carboplatin, ciprofloxacin, fluconazole, folic acid, gemcitabine, granisetron, hydrochlorothiazide, ibandronate, lamivudine, lamotrigine, metformin, metronidazole, niacin, oxaliplatin, oxycodone, parathyroid hormone (PTH), progesterone, ranitidine, risedronate, rosiglitazone, sumatriptan, timolol maleate, and zoledronic acid. In some embodiments of the invention, the hydrophilic drug is bendamustine or gemcitabine. In a certain embodiment of the invention, the hydrophilic drug is present in an amount ranging from about 0.2% to about 15% (w/w) based on the weight of the pharmaceutical composition.

According to the invention, the one or more solvents as used herein are capable of dissolving a given hydrophilic drug or its pharmaceutically acceptable salt to form a drug-solvent solution. Particularly, each of the one or more solvents as used herein is capable of dissolving about 1 part of a given hydrophilic drug or its pharmaceutically acceptable salt in less than 100 parts of the solvent. More particularly, the one or more solvents as used herein may be selected from the group consisting of (a) a first solvent capable of dissolving about 1 part of a given hydrophilic drug or its pharmaceutically acceptable salt in less than about 1 part of the first solvent (very soluble); (b) a second solvent capable of dissolving about 1 part of a given hydrophilic drug or its pharmaceutically acceptable salt in about 1 to about 10 parts of the second solvent (free soluble); (c) a third solvent capable of dissolving about 1 part of a given hydrophilic drug or its pharmaceutically acceptable salt in about 10 to about 30 parts of the third solvent (soluble); (d) a forth solvent capable of dissolving about 1 part of a given hydrophilic drug or its pharmaceutically acceptable salt in about 30 to about 100 parts of the fourth solvent (sparingly soluble); and (e) any combination thereof. Generally, approximate quantity of solvent by volume is for one part of soluble by weight. For example, a first solvent is capable of dissolving about 1g of a given hydrophilic drug in less than 1 ml of the solvent. Examples of the solvent(s) as used herein include but are not limited to water, ethanol, polyethylene glycol (PEG), isopropanol (IPA), 1,2-propanediol (propylene glycol), glycerol, and acetic acid. Any of the solvents can be used alone or in combination. In one embodiment, the self micro-emulsifying pharmaceutical composition of the invention contains water as a solvent. In a certain embodiment of the invention, the one or more solvents are present in an amount ranging from about 2.5% to about 60% (w/w) based on the weight of the pharmaceutical composition.

According to the present invention, the surfactant system comprises one or more surfactants and exhibits a HLB value ranging from about 8 to about 17. A HLB value is known in the art for ranking surfactants according to the balance between the hydrophilic and lipophilic portions of the surfactant agent; the higher the HLB value, the more hydrophilic the surfactant agent; and the lower the HLB value, the less hydrophilic the surfactant agent. One single surfactant having a HLB value ranging from about 8 to about 17 may be used in the present invention. Alternatively, a combination of a high HLB surfactant and a low HLB surfactant may be used; such mixed surfactants are present in a ratio so that the mixture of the surfactants remains to exhibit a final HLB value ranging from about 8 to about 17. The surfactant(s) to be used herein may be cationic surfactants, anionic surfactants, or nonionic surfactants. Examples of the surfactant(s) include but are not limited to polysorbate, poloxamers, oleoyl polyoxylglycerides (such as Labrafil M1944CS), linoleoyl polyoxylglycerides (such as Labrafil M2125CS), caprylocaproyl polyoxylglycerides (such as Labrasol), polyoxyethylene castor oil derivatives (such as PEG 40 hydrogenated castor oil, Cremophor EL or Cremophor RH), polyoxyethylene alkyl ethers (such as Brij), sorbitan fatty acid esters (such as Spans), glyceryl monooleate (such as PECEOL®), glyceryl monolinoleate (such as Maisine® 35-1), medium-chain triglycerides (MCT), polyglyceryl oleate (such as Plurol® Oleique CC497), lauroyl polyoxylglyceride (such as Gelucire® 44/14), stearoyl polyoxylglycerides (such as Gelucire® 50/13), propylene glycol dicaprylocaprate (such as Labrafac® PG), propylene glycol laurate (such as Lauroglycol® FCC), propylene glycol monolaurate (such as Lauroglycol® 90), propylene glycol caprylate (such as Capryol PGMC) and propylene glycol monocaprylate (such as Capryol 90). Any of the surfactants can be used alone or in combination. More preferably, a single surfactant or a combination of surfactants, having a HLB value from about 9 to about 13, ever more preferably from about 10 to about 12, is used. In a certain embodiment, the self-emulsifying pharmaceutical composition of the invention contains polysorbate and oleoyl polyoxylglycerides as the surfactant system. In a certain embodiment of the invention, the surfactant system is present in an amount ranging from about 20.0% to about 75% (w/w) based on the weight of the pharmaceutical composition.

According to the invention, the one or more hydrophilic carriers as used herein are compatible with the above-mentioned drug-solvent solution, composed of the hydrophilic drug and the solvent(s), and said surfactant system. As used herein, the term "compatible" means that the one or more hydrophilic carriers are mixable or dispersed with the above-mentioned drug-solvent solution and the surfactant system so as to form a stable homogenous solution. Particularly, each of the one or more hydrophilic carriers as used herein is capable of dissolving about 1 part of a given hydrophilic drug or its pharmaceutically acceptable salt in about 10 to about 10,000 parts of the hydrophilic carrier. More particularly, the one or more hydrophilic carriers as used herein may be selected from the group consisting of (a) a first hydrophilic carrier capable of dissolving about 1 part of a given hydrophilic drug or its pharmaceutically acceptable salt in about 10 to about 30 parts of the first hydrophilic carriers (soluble); (b) a second hydrophilic carrier capable of dissolving about 1 part of a given hydrophilic drug or its pharmaceutically acceptable salt in about 30 to about 100 parts of the second hydrophilic carrier (sparingly soluble); (c) a third hydrophilic carrier capable of dissolving about 1 part of a given hydrophilic drug or its pharmaceutically acceptable salt in about 100 to about 1,000 parts of the third hydrophilic carrier (slightly soluble); (d) a forth hydrophilic carrier capable of dissolving about 1 part of a given hydrophilic drug or its pharmaceutically acceptable salt in about 1,000 to about 10,000 parts of the fourth hydrophilic carrier (very slightly soluble); and (e) any combination thereof. Examples of the hydrophilic carrier(s) as used herein include but are not limited to polysorbate, ethanol, polyethylene glycol (PEG) such as PEG200, PEG300, PEG400, PEG600, PEG1000, PEG2000, PEG3000, PEG4000, PEG6000, or PEG8000, glycerol, 1,2-propanediol (propylene glycol), propylene carbonate (PC), and diethylene glycol monoethyl ether (such as Transcutol® HP). Any of the hydrophilic carriers can be used alone or in combination. In a certain embodiment of the invention, the one or more hydrophilic carrier are present in an amount ranging from about 2% to about 60% (w/w) based on the weight of the pharmaceutical composition.

Further, in some instances, it may be particularly advantageous to use certain combinations of the solvent(s) and the hydrophilic carrier(s), for example (i) a first solvent in combination with a second, third or fourth hydrophilic carrier, (ii) a second solvent in combination with a second or third hydrophilic carrier, (iii) a third solvent in combination with a second or third hydrophilic carrier, or (iv) a fourth solvent in combination with a first, second or third hydrophilic carrier. In addition, the solvent(s) and the hydrophilic carrier(s) are particularly together present in an amount ranging from about 25% to about 65% (w/w), more particularly about 40% to about 60% (w/w), and even more particularly about 50% (w/w), based on the weight of the pharmaceutical composition of the invention. Specifically, the solvent(s) and the hydrophilic carrier(s) are present at the ratio of about 1: 0.1 to about 1: 9 by weight in the pharmaceutical composition of the invention. More specifically, if the pharmaceutical composition of the invention is in the form of oral solution, the solvent(s) and the hydrophilic carrier(s) are present at the ratio of about 1:0.1 to about 1: 2 by weight in the pharmaceutical composition of the invention; and if the pharmaceutical composition of the invention is in the form of capsule, the solvent(s) and the hydrophilic carrier(s) are present at the ratio of about 1: 1 to about 1: 9 by weight in the pharmaceutical composition of the invention. On the other hand, the hydrophilic carrier(s) and the surfactant system are particularly together present in an amount ranging from about 50% to about 95% (w/w), more particularly about 65% to about 85% (w/w), and even more particularly about 75% (w/w), based on the weight of the pharmaceutical composition of the invention. Specifically, the hydrophilic carrier(s) and the surfactant system are present at the ratio of about 1: 0.3 to about 1: 32.5, more specifically about 1: 1 to about 1:20, and even more specifically about 1: 1.5 by weight in the pharmaceutical composition of the invention.

In one embodiment, the solvent(s), the hydrophilic carrier(s) and the surfactant system are present at the ratio of about 2: 3:4.5 by weight in the pharmaceutical composition of the invention.

In addition, the self micro-emulsifying pharmaceutical composition of the invention may optionally include other components such as an antioxidant e.g. D-$\alpha$-tocopheryl polyethylene glycol 1000 succinate (TPGS).

In a certain embodiment, the self micro-emulsifying pharmaceutical composition of the invention comprises gemcitabine or its pharmaceutically acceptable salt, water, glycerol, PEG, polysorbate, and oleoyl polyoxylglycerides. In a specific example, gemcitabine is present in an amount of about 2.00% (w/w) based on the weight of the pharmaceutical composition; water is present in an amount of about 20.00% (w/w) based on the weight of the pharmaceutical composition; glycerol and PEG are together present in an amount of about 32.30% (w/w) based on the weight of the pharmaceutical composition; and polysorbate, and oleoyl polyoxylglycerides are together present in an amount of about 45.70% (w/w) based on the weight of the pharmaceutical composition.

In a certain embodiment, the self-emulsifying pharmaceutical composition of the invention comprises gemcitabine or its pharmaceutically acceptable salt, water, propylene glycol, PEG, polysorbate, and oleoyl polyoxylglycerides. In a specific example, gemcitabine is present in an amount of about 2.00% (w/w) based on the weight of the pharmaceutical composition; water is present in an amount of about 20.00% (w/w) based on the weight of the pharmaceutical composition; propylene glycol and PEG are together present in an amount of about 32.30% (w/w) based on the weight of the pharmaceutical composition; and polysorbate, and oleoyl polyoxylglycerides are together present in an amount of about 45.70% (w/w) based on the weight of the pharmaceutical composition.

In a certain embodiment, the self-emulsifying pharmaceutical composition of the invention comprises gemcitabine or its pharmaceutically acceptable salt, water, glycerol, PEG, polysorbate, oleoyl polyoxylglycerides, and TPGS. In a specific example, gemcitabine is present in an amount of about 1.98% (w/w) based on the weight of the pharmaceutical composition; water is present in an amount of about 19.8% (w/w) based on the weight of the pharmaceutical composition; glycerol and PEG are together present in an amount of about 31.98% (w/w) based on the weight of the pharmaceutical composition; polysorbate and oleoyl polyoxylglycerides are together present in an amount of about 45.25% (w/w) based on the weight of the pharmaceutical composition; and TPGS is present in an amount of about 0.99% (w/w) based on the weight of the pharmaceutical composition.

In addition, the self micro-emulsifying pharmaceutical composition of the invention is optionally adjusted to have a pH above the dissociation constant (pKa) of the hydrophilic drug contained therein to increase stability during storage. In one embodiment, the self micro-emulsifying pharmaceutical composition of the invention containing gemcitabine is further adjusted to have a pH above 4.0, e.g., at pH 4-5, 5-6, 6-7, or 7-8.

The self micro-emulsifying pharmaceutical composition according to the invention exhibits excellent bioavailability of the drug through oral administration which is comparable to that of the drug through intravenous injection. In a specific example, the self-emulsifying pharmaceutical composition according to the invention shows relative bioavailability of about 89% through oral administration as compared to the conventional formulation through injection (see Example 4 below).

Further, the self micro-emulsifying pharmaceutical composition according to the invention also exhibits good stability during storage, which particularly means that there is no substantial phase separation, material precipitation, texture change, or degradation of an active ingredient contained therein during a certain storage period. The term "no substantial degradation of an active ingredient contained therein" means that the amount of the active ingredient lost in the pharmaceutical composition of the invention after being stored for a certain period of time is less than 20%, preferably less than 10%, and more preferably less than about 5 % of the original amount of the active ingredient in the pharmaceutical composition.

The pharmaceutical compositions of this invention may be orally administered in any orally acceptable dosage form including, but not limited to, capsules, tablets, powders or coated granules, which may contain pharmaceutical excipients known in the art such as binders, fillers, filler/binders, adsorbents, moistening agents, disintegrants, lubricants and the like as needed.

In certain embodiments of the invention, the pharmaceutical composition is encapsulated in a sealed soft or hard capsule. The capsule is typically of a kind which is dissolved in a particular region of the GI tract releasing its content there. An example of such a capsule is an enteric-coated soft or hard gelatin capsule. Enteric coating, as known *per se,* is coating with a substance or a combination of substances that resists dissolution in gastric fluid but disintegrates in the intestine.

The pharmaceutical composition of the present invention can be prepared by mixing the hydrophilic drug with the one or more solvents, the one or more hydrophilic carriers, and the surfactant system using any standard method commonly used in the art in view of the present disclosure. in the examples below.

The present invention will now be described more specifically with reference to the following embodiments, which are provided for the purpose of demonstration rather than limitation.

**Example 1: Preparation of self emulsifying pharmaceutical compositions of the invention**

1. Formulation I

Gemcitabine hydrochloride (100 mg) was added to distilled water (1,000 mg), glycerol (105 mg) and PEG 400 (1,510 mg) and agitated until completely dissolved to form Solution A. Tween 80 (1,613 mg) and Labrafil M1944CS (672mg) were homogenously mixed in another container to form Solution B. Solution A was then poured into Solution B, and agitated until a clear solution was obtained to form Formulation I, which was further made into a hard/soft capsule using a well-known method in the art.

Table 1 shows the composition of Formulation I.

Table 1

|  | Component | weight (mg) | percentage (%) |
| --- | --- | --- | --- |
| Formulation I | gemcitabine HCl | 100 | 2.00 |
| pH 1-2 | Water | 1,000 | 20.00 |
| HLB of surfactants | glycerol I | 105 | 2.10 |
| (11.76) | PEG 400 | 1,510 | 30.20 |
|  | Tween 80 | 1,613 | 32.30 |
|  | Labrafi M1944 CS | 672 | 13.40 |
|  | Total | 5,000 | 100.00 |

2. Formulation II

First, gemcitabine hydrochloride (100 mg) was added to distilled water (1,000 mg), propylene glycol (105 mg) and PEG 400 (1,510 mg) and agitated until completely dissolved to form Solution A. Tween 80 (1,613 mg) and Labrafil M1944CS (672 mg) were homogenously mixed in another container to form Solution B. Solution A was then poured into Solution B and agitated until a clear solution was obtained to form Formulation 11 which was further made into a hard/soft capsule using a well-known method in the art.

Table 2 shows the composition of Formulation II.

Table 2

|  | component | weight (mg) | Percentage (%) |
| --- | --- | --- | --- |
| Formulation II | gemcitabine HCl | 100 | 2.00 |
| pH 1-2 | water | 1,000 | 20.00 |
| HLB of surfactants | propylene glycol | 105 | 2.10 |

(continued)

| | component | weight (mg) | Percentage (%) |
|---|---|---|---|
| (11.76) | PEG 400 | 1,510 | 30.20 |
| | Tween 80 | 1,613 | 32.30 |
| | Labrafil M 1944 CS | 672 | 13.40 |
| | Total | 5,000 | 100.00 |

3. Formulation III

Gemcitabine hydrochloride (100 mg) was added to distilled water (1,000 mg), glycerol (105 mg), PEG 400 (1,510 mg) and TPGS (50 mg), and agitated until completely dissolved to form Solution A. Tween 80 (1,613 mg) and Labrafil M1944CS (672 mg) were homogenously mixed in another container to form Solution B. Solution A was then poured into Solution B and agitated until a clear solution was obtained to form Formulation III which was further made into a hard/soft capsule using a well-known method in the art. Table 3 shows the composition of Formulation III.

Table 3

| | component | weight (mg) | Percentage (%) |
|---|---|---|---|
| Formulation III | gemcitabine HCl | 100 | 1.98 |
| pH 1-2 | Water | 1,000 | 19.80 |
| HLB of surfactants | glycerol | 105 | 2.08 |
| (11.76) | PEG 400 | 1,510 | 29.90 |
| | TPGS | 50 | 0.99 |
| | Tween 80 | 1,613 | 31.94 |
| | Labrafil M1944 CS | 672 | 13.31 |
| | Total | 5,050 | 100.00 |

4. Formulation IV

Gemcitabine hydrochloride (100 mg) was added into distilled water (901.3mg), 4.0 N NaOH solution (98.7 mg) glycerol (105 mg), PEG 400 (1,510 mg), and agitated until completely dissolved to form Solution A. Tween 80 (1,613mg) and Labrafil M1944CS (672mg) were homogenously mixed in another container to form Solution B. Solution A was then poured into Solution B, and agitated until a clear solution was obtained to form Formulation IV which was further made into a hard/soft capsule using a well-known method in the art. Table 4 shows the composition of Formulation IV.

Table 4

| | component | weight (mg) | Percentage (%) |
|---|---|---|---|
| Formulation IV | gemcitabine HCl | 100 | 2.00 |
| pH 5-6 | water | 901.3 | 18.03 |
| HLB of surfactants | 4.0 N NaOH | 98.7 | 1.97 |
| (11.76) | glycerol | 105 | 2.10 |
| | PEG 400 | 1,510 | 30.20 |
| | Tween 80 | 1,613 | 32.30 |
| | Labrafil M 1944 CS | 672 | 13.40 |
| | Total | 5,000 | 100.00 |

5. Formulation V

Gemcitabine hydrochloride (100 mg) was added to distilled water (901.3 mg), 4.0 N NaOH solution (98.7mg), propylene glycol (105 mg), PEG 400 (1,510mg), TPGS (50mg), and agitated until completely dissolved to form Solution A. Tween 80 (1,613mg) and Labrafil M 1944CS (672mg) were homogenously mixed in another container to form Solution B. Solution A was then poured into Solution B, and agitated until a clear solution was obtained to form Formulation V which was further made into a hard/soft capsule using a well-know method in the art. Table 5 shows the composition of Formulation V.

Table 5

|  | component | weight (mg) | Percentage (%) |
|---|---|---|---|
| Formulation V | gemcitabine HCl | 100 | 1.98 |
| pH 5-6 | water | 901.3 | 17.85 |
| HLB of surfactants | 4.0 N NaOH | 98.7 | 1.95 |
| (11.76) | propylene Glycol | 105 | 2.08 |
|  | PEG 400 | 1,510 | 29.90 |
|  | TPGS | 50 | 0.99 |
|  | Tween 80 | 1,613 | 31.94 |
|  | Labrafil M1944 CS | 672 | 13.31 |
|  | Total | 5050 | 100 |

6. Formulation VI

Gemcitabine hydrochloride (100 mg) was added to distilled water (913.28 mg), 4.0 N NaOH solution (86.72 mg), glycerol (105 mg), and PEG 400 (1,510 mg), and agitated until completely dissolved to form Solution A. Tween 80 (1,613 mg) and Labrafil M1944CS (672 mg) were homogenously mixed in another container to form Solution B. Solution A was then poured into Solution B, and agitated until a clear solution was obtained to form Formulation VI which was further made into a hard/soft capsule using a well-know method in the art. Table 6 shows the composition of Formulation VI.

Table 6

|  | component | weight (mg) | Percentage (%) |
|---|---|---|---|
| Formulation VI | gemcitabine HCl | 100 | 2.00 |
| pH 4-5 | water | 913.28 | 18.26 |
| HLB of surfactants | 4.0 N NaOH | 86.72 | 1.74 |
| (11.76) | glycerol | 105 | 2.10 |
|  | PEG 400 | 1,510 | 30.20 |
|  | Tween 80 | 1,613 | 32.30 |
|  | Labrafil M 1944 CS | 672 | 13.40 |
|  | Total | 5000 | 100 |

7. Formulation VII

Gemcitabine hydrochloride (100 mg) was added to distilled water (720.21 mg), 4.0 N NaOH solution (279.79 mg), glycerol (105 mg), and PEG 400 (1,510 mg), and agitated until completely dissolved to form Solution A. Tween 80 (1,613 mg) and Labrafil M1944CS (672 mg) were homogenously mixed in another container to form Solution B. Solution A was then poured into Solution B, and agitated until a clear solution was obtained to form Formulation VII which was further made into a hard/soft capsule using a well-know method in the art.

Table 7 shows the composition of Formulation VII.

Table 7

|  | component | weight (mg) | Percentage (%) |
|---|---|---|---|
| Formulation VII | gemcitabine HCl | 100 | 2.00 |
| pH 6-7 | water | 720.21 | 14.40 |
| HLB of surfactants | 4.0 N NaOH | 279.79 | 5.60 |
| (11.76) | glycerol | 105 | 2.10 |
|  | PEG 400 | 1,510 | 30.20 |
|  | Tween 80 | 1,613 | 32.30 |
|  | Labrafil M 1944 CS | 672 | 13.40 |
|  | Total | 5000 | 100 |

8. Formulation VIII

Gemcitabine hydrochloride (100 mg) was added to distilled water (715 mg), 4.0 N NaOH solution (285 mg), glycerol (105 mg), and PEG 400 (1,510 mg), and agitated until completely dissolved to form Solution A. Tween 80 (1,613 mg) and Labrafil M1944CS (672 mg) were homogenously mixed in another container to form Solution B. Solution A was then poured into Solution B, and agitated until a clear solution was obtained to form Formulation VIII which was further made into a hard/soft capsule using a well-know method in the art.

Table 8 shows the composition of Formulation VIII.

Table 8

|  | component | weight (mg) | Percentage (%) |
| --- | --- | --- | --- |
| Formulation VIII | Gemcitabine HCl | 100 | 2.00 |
| pH 7-8 | water | 715 | 14.30 |
| HLB of surfactants | 4.0 N NaOH | 285 | 5.70 |
| (11.76) | glycerol | 105 | 2.10 |
|  | PEG 400 | 1,510 | 30.20 |
|  | Tween 80 | 1,613 | 32.30 |
|  | Labrafil M1944 CS | 672 | 13.40 |
|  | Total | 5000 | 100.00 |

**Example 2: Measurement of particle size of self-emulsifying pharmaceutical compositions of the invention**

The particle size of the microemulsion droplets of Formulations I to VIII was measured. Briefly, 250 ml distilled water was poured into the dissolution mini vessel and heated to 37°C. Once the temperature reached 37°C, 0.25 ml of the formulation to be tested was added into the vessel. The mixture was agitated by paddle at 100 rpm for 10 minutes. After 10 minutes, transferred about 1 ml mixture to a sample cuvette, then measured the particle size of microemulsion droplets by Zetasizer (Zetasizer Nano-ZS,, Malvern Inst., UK) which following the instructions given in the manuals provided by the manufacturer. Table 9 shows the particle sizes of the microemulsions formed by the pharmaceutical compositions of the present invention as measured.

Table 9

| Droplet Particle Sizes (Z-average: d. nm) | |
| --- | --- |
| Formulation I | 10.13 |
| Formulation II | 9.57 |
| Formulation III | 12.65 |
| Formulation IV | 13.35 |
| Formulation V | 16.15 |
| Formulation VI | 64.58 |
| Formulation VII | 89.45 |
| Formulation VIII | 83.18 |

**Example 3: Preparation of a comparative formulation for injection**

Gemcitabine hydrochloride (53 mg) was added into a normal saline (4,947 mg), and agitated until completely dissolved to form a comparative formulation (5000 mg). Table 10 shows the composition of the comparative formulation.

Table 10

|  | component | weight (mg) | percentage (%) |
| --- | --- | --- | --- |
| Comparative formulation | gemcitabine HCl | 53 | 1.06% |
| (powder, intravenous injection dosage form) | water | 4947 | 98.94% |
|  | Total | 5,000 | 100.00% |

**Example 4: Bioassay**

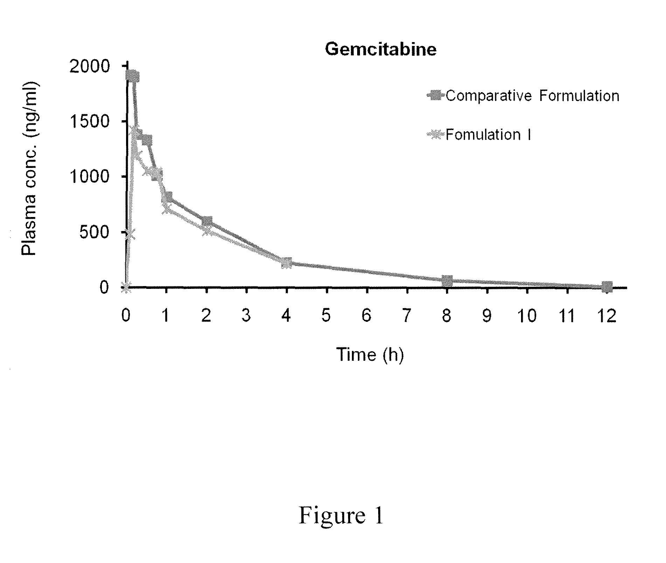

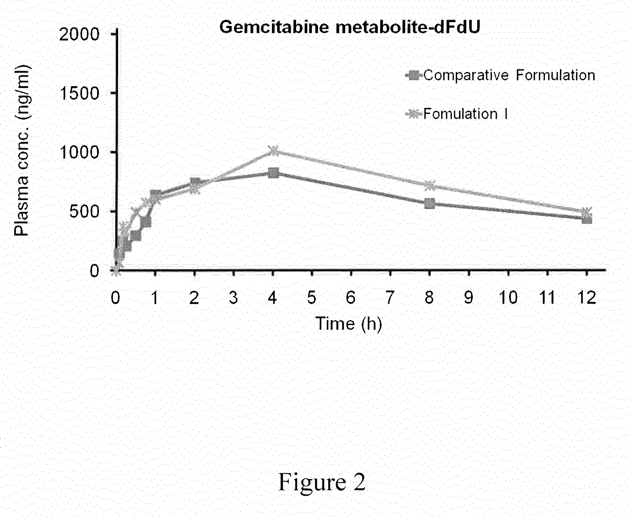

Formulation I (1 mg/kg) as prepared in Example 1 were administrated to a beagle dog via feeding tube; and the comparative formulation (1 mg/kg) as prepared in Example 3 was administrated to another beagle dog by intravenous injection. The blood of the dogs was collected at 5, 10, 15, 30, and 45 minutes, and 1, 2, 4, 8, and 12 hours after the administration, respectively. The collected blood was added into a tube with a reaction terminator and an anticoagulant, and the mixture was subsequently centrifuged to obtain the plasma. Gemcitabine and its main metabolite were analyzed by LC/MS/MS (liquid chromatography/ mass spectrometer). Figures 1 and 2 and Tables 11 and 12 shows the results of the bioassay.

Table 11: Non-compartment model analysis of plasma gemcitabine pharmacokinetic parameters

| Route | i.v. injection | oral administration |
|---|---|---|
| Formulations (1 mg/kg) | Comparative Formulation | Formulation I |
| $AUC_{0-t}$ (mg*h/L) | 3.57 | 2.62 |
| $AUC_{0-\infty}$ (mg*h/L) | 3.60 | 3.22 |
| $C_{max}$(mg/L) | 1.92 | 1.62 |
| Tmax(h) | 0.08 | 0.17 |
| $T_{1/2}$(h) | 1.79 | 1.71 |

Table 12: Non-compartment model analysis of plasma dFdU pharmacokinetic parameters

| Route | i.v. injection | oral administration |
|---|---|---|
| Formulation (1 mg/kg) | Comparative Formulation | Formulation I |
| $AUC_{0-t}$ (mg*h/L) | 7.26 | 9.43 |
| $AUC_{0-\infty}$ (mg*h/L) | 12.81 | 17.08 |
| $C_{max}$(mg/L) | 0.82 | 1.01 |
| $T_{max}$(h) | 4.00 | 4.00 |
| $T_{1/2}$(h) | 8.77 | 9.44 |

The results show that gemcitabine can be well absorbed in the animals through oral administration of the self micro-emulsifying pharmaceutical composition of the invention. The relative bioavailability of the self micro-emulsifying pharmaceutical composition of the invention is about 89% (3.22/3.60) as compared to the comparative formulation through i.v. injection. Also, the plasma profile of dFdU of the self micro-emulsifying pharmaceutical composition of the invention is similar to that of the comparative formation, suggesting less first-pass metabolic effects compared to that of other oral formulations of gemcitabine in the prior art. The present invention for the first time provides a self micro-emulsifying pharmaceutical composition of gemcitabine with comparable bioavailability to that of conventional formulations through i.v. injection as used in the art.

**Example 4: Stability test method**

Formulations I to VIII of Example 1 were subjected to a stability test which can be conducted based on a conventional method known in the art. Briefly, about 2 g of the formulation was added into a vial (4 ml) which was then filled with nitrogen and sealed with Teflon septum and aluminum cap. The sealed vials were subsequently put in a Constant Temperature and Humidity Chamber (25°C 60% RH or 40°C 75%RH) for at least 30 days. On each time point, some of the vials were taken out and the samples inside were poured into a volumetric flask (100 ml). Residual samples were eluted with distilled water and collected in the flask as well. The flask was finally filled with water to 100 ml. HPLC analysis was then conducted to determine the amount (w) of gemcitabine in the samples collected in the flask. The degradation rate (%) of gemcitabine is calculated as below:

$$1 - \frac{\text{Amount of the hydrophilic drug at Day 7, 14, 21 or 30}}{\text{Amount of the hydrophilic drug at Day 0}} \times 100\%$$

Table 13 shows the results of the degradation rate of Formulations I to VIII of the invention.

| | | Formulations | | | | | | | |
|---|---|---|---|---|---|---|---|---|---|
| | | I (pH 1-2) | II (pH 1-2) | III (pH 1-2) | IV (pH 5-6) | V (pH 5-6) | VI (pH 4-5) | VII (pH 6-7) | VIII (pH 7-8) |
| Time | Condition | degradation rate (%) | | | | | | | |
| 7 day | 1 | 2.80% | 3.73% | 3.79% | 4.09% | 3.86% | 6.12% | 4.16% | 4.75% |
| | 2 | 4.64% | 5.39% | 5.67% | 4.60% | 3.62% | 4.11% | 3.73% | 4.97% |
| 14 day | 1 | 2.79% | 1.48% | 2.29% | 3.61% | 4.90% | 5.21% | 6.92% | 5.73% |
| | 2 | 6.53% | 7.59% | 5.76% | 4.02% | 2.32% | 4.77% | 6.06% | 6.24% |
| 21 day | 1 | 5.37% | 6.76% | 9.83% | 4.35% | 7.95% | ND | ND | ND |
| | 2 | 14.80% | 10.21% | 12.71% | 6.25% | 8.76% | ND | ND | ND |
| 30 day | 1 | 5.80% | 5.82% | 6.31% | 4.48% | 4.67% | 5.51% | 5.17% | 4.75% |
| | 2 | 13.61% | 12.39% | 12.60% | 5.19% | 6.75% | 5.96% | 4.26% | 6.56% |

Condition 1 is 25°C and 60% relative humility.
Condition 2 is 40°C and 75% relative humility.
ND means not determined.

According to the results, Formulations I to VIII of the invention exhibit high stability at room temperature (25°C) for at least 30 days (less than 10% of the degradation rate), and among them Formulations IV to VIII (pH above 4) exhibit high stability at 40 °C for at least 30 days (less than 10% of the degradation rate).

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features. From the above description, one skilled in the art can make various changes and modifications of the invention to adapt it to various usages and conditions without departing from the spirit and scope thereof. Therefore, this invention is not limited to the specific embodiments described herein, and the right is reserved to the illustrated embodiments and all modifications coming within the scope of the following claims.

**Claims**

1.  An oral self micro-emulsifying pharmaceutical composition, which comprises:

    (a) a therapeutically effective amount of a hydrophilic drug or its pharmaceutically acceptable salt;
    (b) one or more solvents capable of dissolving the hydrophilic drug or its pharmaceutically acceptable salt to form a drug-solvent solution;
    (c) a surfactant system comprising one or more surfactants, said surfactant system exhibiting a hydrophilic-lipophilic balance (HLB) value ranging from about 8 to about 17; and
    (d) one or more hydrophilic carriers which are compatible with said drug-solvent solution and said surfactant system;

    wherein the pharmaceutical composition is in a form of a self micro-emulsifying formulation for oral administration.

2.  The oral self micro-emulsifying pharmaceutical composition of Claim 1, which forms an emulsion with a particle size of less than about 800 nm when said pharmaceutical composition contacts an aqueous medium.

3.  The oral self micro-emulsifying pharmaceutical composition of Claim 1, wherein the hydrophilic drug is bendamustine or gemcitabine.

4.  The oral self micro-emulsifying pharmaceutical composition of Claim 1, wherein each of the one or more solvents is capable of dissolving about 1 part of a given hydrophilic drug or its pharmaceutically acceptable salt in less than 100 parts of the solvent.

5.  The oral self micro-emulsifying pharmaceutical composition of Claim 1, wherein the one or more solvents are selected from the group consisting of water, ethanol, polyethylene glycol (PEG), isopropanol (IPA), 1,2-propanediol (propylene glycol), glycerol, acetic acid, and any combinations thereof.

6. The oral self micro-emulsifying pharmaceutical composition of Claim 5, which comprises water as the solvent.

7. The oral self micro-emulsifying pharmaceutical composition of Claim 1, wherein the one or more surfactants are selected from the group consisting of polysorbate, poloxamers, oleoyl polyoxylglycerides, linoleoyl polyoxylglycerides, caprylocaproyl polyoxylglycerides, polyoxyethylene castor oil derivatives, polyoxyethylene alkyl ethers, sorbitan fatty acid esters, glyceryl monooleate, glyceryl monolinoleate, medium-chain triglycerides (MCT), polyglyceryl oleate, lauroyl polyoxylglyceride, stearoyl polyoxylglycerides, propylene glycol dicaprylocaprate, propylene glycol laurate, propylene glycol monolaurate, propylene glycol caprylate and propylene glycol monocaprylate, and combinations thereof.

8. The oral self micro-emulsifying pharmaceutical composition of Claim 7, which comprise polysorbate and oleoyl polyoxylglycerides as the surfactants.

9. The oral self micro-emulsifying pharmaceutical composition of Claim 1, wherein each of the one or more hydrophilic carriers is capable of dissolving about 1 part of a given hydrophilic drug or its pharmaceutically acceptable salt in about 10 to about 10,000 parts of the hydrophilic carrier.

10. The oral self micro-emulsifying pharmaceutical composition of Claim 1, wherein the one or more hydrophilic carriers are selected from the group consisting of polysorbate, ethanol, polyethylene glycol (PEG), glycerol, 1,2-propanediol (propylene glycol), propylene carbonate (PC), diethylene glycol monoethyl ether, and combinations thereof.

11. The oral self micro-emulsifying pharmaceutical composition of Claim 10, which comprises glycerol and PEG as the hydrophilic carriers.

12. The oral self micro-emulsifying pharmaceutical composition of Claim 10, which comprises propylene glycol and PEG as the hydrophilic carriers.

13. The oral self micro-emulsifying pharmaceutical composition of Claim 3, which has a pH above the 4.0.

14. The oral self micro-emulsifying pharmaceutical composition of Claim 1, which comprises gemcitabine or its pharmaceutically acceptable salt, water, glycerol, PEG, polysorbate, and oleoyl polyoxylglycerides.

15. The oral self micro-emulsifying pharmaceutical composition of Claim 1, which comprises gemcitabine or its pharmaceutically acceptable salt, water, propylene glycol, PEG, polysorbate, and oleoyl polyoxylglycerides.

16. The oral self micro-emulsifying pharmaceutical composition of Claim 1, which comprises gemcitabine or its pharmaceutically acceptable salt, water, glycerol, PEG, polysorbate, oleoyl polyoxylglycerides, and TPGS.

17. A method for preparing an oral self micro-emulsifying pharmaceutical composition of claim 1, comprising mixing together the hydrophilic drug or its pharmaceutically acceptable salt thereof, the one or more solvents, the one or more hydrophilic carriers and the surfactant system to form the oral self micro-emulsifying pharmaceutical composition..

18. The method of clam 17, comprising mixing the hydrophilic drug or its pharmaceutically acceptable salt thereof with the one or more solvents and the one or more hydrophilic carriers first and further with the surfactant system.

Figure 1

Figure 2

# INTERNATIONAL SEARCH REPORT

| International application No. |
| --- |
| PCT/CN2010/000577 |

### A. CLASSIFICATION OF SUBJECT MATTER

See extra sheet

According to International Patent Classification (IPC) or to both national classification and IPC

### B. FIELDS SEARCHED

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

DATABASE: WPI, EPODOC, CNPAT, CNKI, CA, EMBASE

KEY WORDS: self-emulsifying, self-emulsion, self-microemulsifying, self-emulsion, emulsion, hydrophilic, surfactant, surface active agent, buspirone, calcitonin, captopril, carboplatin, ciprofloxacin, fluconazole, gemcitabine, granisetron, hydrochlorothiazide, ibandronate, lamivudine, lamotrigine, metronidazole, niacin, oxaliplatin, oxycodone, progesterone, ranitidine, risedronate, rosiglitazone, sumatriptan, SEDDS, SMEDDS, bendamustine, gemcitabine, zoledronic acid, timolol maleate, parathyroid hormone, PTH, folic acid

### C. DOCUMENTS CONSIDERED TO BE RELEVANT

| Category* | Citation of document, with indication, where appropriate, of the relevant passages | Relevant to claim No. |
| --- | --- | --- |
| X | CN 1593405A(UNIVERSITY SHENYANG PHARMACEUTICAL), 16 March 2005(16.03.2005), see claims 1-6, page 1 lines 33-34, example 12 | 1, 2, 4-12, 17-18 |
| A | | 3, 13-16 |
| A | CN 1791383A(ADVANCED MEDICAL OPTICS INC.)，21 June 2006(21.06.2006), see the whole document | 1-18 |

☐ Further documents are listed in the continuation of Box C.  ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim (S) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&"document member of the same patent family |

| Date of the actual completion of the international search | Date of mailing of the international search report |
| --- | --- |
| 07 July 2010(07.07.2010) | **05 Aug. 2010 (05.08.2010)** |
| Name and mailing address of the ISA/CN<br>The State Intellectual Property Office, the P.R.China<br>6 Xitucheng Rd., Jimen Bridge, Haidian District, Beijing, China 100088<br>Facsimile No. 86-10-62019451 | Authorized officer<br>LI, Fengyun<br>Telephone No. (86-10)62411143 |

Form PCT/ISA /210 (second sheet) (July 2009)

**INTERNATIONAL SEARCH REPORT**
Information on patent family members

| International application No. |
|---|
| *PCT/CN2010/000577* |

| Patent Documents referred in the Report | Publication Date | Patent Family | Publication Date |
|---|---|---|---|
| CN 1593405A | 16.03.2005 | NONE | |
| CN 1791383A | 21.06.2006 | US 2004185068A1 | 23.09.2004 |
| | | WO 2004082625A2 | 30.09.2004 |
| | | EP 1603607A2 | 14.12.2005 |
| | | AU 2004222295A1 | 30.09.2004 |
| | | BRPI 0408516A | 07.03.2006 |
| | | JP 2006526579 T | 24.11.2006 |
| | | AU 2004222295 B2 | 17.07.2008 |

Form PCT/ISA /210 (patent family annex) (July 2009)

## INTERNATIONAL SEARCH REPORT

International application No.

PCT/CN2010/000577

Continuation of classification of subject matter:

A61K 9/107 (2006.01)i

A61K 31/4184 (2006.01)i

A61K 31/7068 (2006.01)i

A61P35/00 (2006.01)i

Form PCT/ISA /210 (extra sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7220428 B
- US 7053076 B
- US 7217735 B
- US 7309696 B
- WO 20040179 A
- WO 2007089043 A